# EUROPEAN PATENT SPECIFICATION

(11) **EP 1 928 422 B1**
(45) Date of publication and mention of the grant of the patent: **25.10.2017**
(21) Application number: 06779620.1
(22) Date of filing: 31.08.2006
(51) Int. Cl.: A61K 9/16

(54) **IMPROVEMENTS IN THE STABILISATION OF BIOLOGICAL MATERIALS**
VERBESSERUNGEN DER STABILISIERUNG VON BIOLOGISCHEN MATERIALIEN
AMELIORATIONS APPORTEES A LA STABILISATION DE MATERIAUX BIOLOGIQUES

(30) Priority: 31.08.2005 GB 0517688
(43) Date of publication of application: 11.06.2008
(73) Proprietor: Nova Bio-Pharma Technologies Limited, Wigston Leicester LE18 4YL (GB)
(72) Inventor: ROSER, Bruce, Cambridgeshire CB1 4OH (GB); MOSS, David, Hertfordshire SG5 4EF (GB)
(74) Representative: Serjeants LLP
(86) International application number: PCT/GB2006/050266
(87) International publication number: WO 2007/026180

(56) References cited:
- EP-A- 0 913 178
- WO-A2-00/72827
- AU-A1- 2005 203 369
- GB-A- 2 413 075
- US-A1- 2003 180 283
- US-A1- 2003 202 978
- US-B1- 6 630 169
- HUTTER W ET AL: "SPRAY DRYING OF THE DEHALOGENATING BACTERIUM RHODOCOCCUS SP" BIOPROCESS ENGINEERING, SPRINGER VERLAG, DE, vol. 13, no. 1, 1995, pages 19-21, XP000601224 ISSN: 0178-515X
- POURRAT H ET AL: "STABILIZATION OF OCTASTATIN, A SOMATOSTATIN ANALOGUE PREPARATION OF FREEZE-DRIED PRODUCTS FOR PARENTERAL INJECTION" BIOLOGICAL & PHARMACEUTICAL BULLETIN (OF JAPAN), PHARMACEUTICAL SOCIETY OF JAPAN, TOKYO, JP, vol. 18, no. 5, 1 May 1995 (1995-05-01), pages 766-771, XP000511937 ISSN: 0918-6158

## Description

This invention relates to the use of glassy substances to stabilise biological materials.

It is known that certain materials, particularly certain sugars, are capable of forming a glass ie a non-crystalline solid which will stabilise biological material carried by it either in suspension or solid solution. Examples of such biological materials are vaccines and insulin. Sometimes the biological material needs to be presented in liquid form e. g. for injection into a patient. For this purpose it has been proposed to form the glassy material as particles suspended in an inert, non-toxic liquid.

A major problem associated with the above proposal has been the selection of a suitable liquid. Traditional liquid carriers used for injection of medications, are oils which are less dense than the sugar glasses usually used for stabilisation causing the latter to sink to the bottom of the liquid. This sediment can become very compact with time. Although the container could be re-agitated to re-form the suspension before administration this agitation may need to be very vigorous and prolonged to be effective. There is an unacceptable risk that such agitation might not be performed properly, thus compromising the effectiveness of the medication.

More recently it has been proposed to use perfluorocarbons as the liquid carrier. Perfluorocarbons have a high density, allowing one to match the density of the particles to that of the liquid by adding a more dense material such as calcium phosphate into the glass. However, although this technique has been shown to work well and is suitable for small-scale or emergency purposes, the use of perfluorocarbons in large quantities needs to be undertaken with caution because it could have an undesired effect on an upper part of the earth's atmosphere. These materials are extremely stable even to high fluxes of ultraviolet light and persist in the stratosphere for millennia where they can constitute a global warming hazard.

According to the present invention there is provided a method of making a glassy product for stabilising a biological material comprising the steps of:
i) mixing (a) a first liquid material comprising a biological material and capable of forming a glass in which the first liquid material comprises a glutamic acid or salt thereof, with (b) a second material which is a blowing agent that undergoes decomposition and forms a gas when heated, in which the second material comprises ammonium bicarbonate; and
ii) causing the first material to form a glass at the same time as the second material forms the gas;
whereby a glassy structure in the form of particles containing the gas is formed in a spray drier.

There is a very wide variety of glassy materials that can be used. Suitable materials include sugars such as raffinose and irehalose, palatinit (a mixture of glucopyranosyl sorbitol and glucopyranosyl mannitol), glucopyranosyl sorbitol, glucopyranosyl mannitol, lactitol, and monosaccharide alcohols.

A problem associated with the use of sugar glasses to stabilise biological materials is that it is necessary to eliminate almost all of the water from the glass to achieve the desired stabilisation effect. Achieving this can require conditions (e.g. a high temperature) which are not compatible with those required to achieve the glassy state and to avoid damaging the active biological material. We have now discovered that this problem can be solved by using glutamic acid or a salt thereof such as monosodium glutamate (hereinafter referred to as "MSG") as a component in the glass forming material, instead of sugars.

MSG has previously been recognised as having stabilising properties when mixed with other glass-forming substances as described for example in patent specification US6872357 and in the paper *"*Recoveries of bacteria after drying in Glutamate and other substances" by D. I. Annear, published in the Aust. J. exp. Biol. Med. Sci (1964), 42. pp. 71 7-722. However, we have now discovered that MSG can have over 3% of residual moisture and up to about 5% yet still be substantially un- softened by it and retain its stabilising properties up to about 70 C. Furthermore, whilst entering the glassy state, we have found that MSG has a pronounced transitional state where it is in the form of a viscous syrup-like semi-solid. This makes it particularly effective for trapping bubbles of gas as they are formed

Where glutamic acid or a salt such as MSG is used it has been found that the active material (particularly if it includes a microparticulate adjuvant) can cause crystallisation to occur. This difficulty can be overcome by including a crystallisation inhibitor such as aspartic acid or a salt thereof. This crystallisation inhibitor is preferably itself a glass former such as monosodium aspartate (MSA) and the two components are preferably present in similar molar ratios (between 4:6 and 6:4) to give optimum inhibition of crystallisation.

During the process of drying a solution of glass-forming material by heat, especially if the drying is taking place in droplets of the material, the temperature of the droplets is kept from rising by a rapid rate of evaporation from the surface of the droplet with its associated evaporative cooling. As the solution becomes more viscous the mobility of water molecules towards the surface of the droplet is slowed by the increasing viscosity and the temperature of the droplet rises as evaporative cooling reduces. Thus the temperature of the drying syrupy droplet rises rapidly as it begins to solidify as a glass.

The gas is introduced into the glass by mixing the glass-forming material (and the biological material) with a chemical that decomposes under the appropriate conditions to form the gas. According to the present invention, the chemical is ammonium bicarbonate which is chosen so that the gas is released when the temperature has risen to a level at which the glass- forming material is in a viscous transitional state between liquid and solid. Ammonium bicarbonate has been identified as a suitable additive because this decomposes into ammonia gas, carbon dioxide and water vapour at about the same temperature as the increase in viscosity i.e. at about 60 C.

The use of MSG is thus particularly appropriate because the water by-product does not adversely affect its stabilising properties. It may be possible; however, to use other glass forming materials such as sugars including raflinose and trebalose. The use of calcium phosphate is of particular interest because of its physical strength. It is conjectured that a solid honeycomb-like component produced in this way could be moulded so as to form a structural part, especially if formed with a continuous outer surface. It would have great strength and light weight similar to the characteristics of animal bone. Possibly such a substance could be used for bone repair or replacement purposes.

Examples of how the invention has been implemented will now be described by way of example with reference to the accompanying illustrations in which: -
Fig 1 is a picture produced by a scanning electron microscope, of glassy particles of MSG produced by a method in accordance with the invention;
Fig 2 is a very schematic drawing showing the particles of Fig 1 suspended in a non-toxic non-aqueous biocompatible liquid to form a stable suspension suitable for injection through a hypodermic syringe; and
Fig 3 shows the results of experiments demonstrating how density of the particles varies with varying ammonium bicarbonate concentrations for different spray drier inlet temperatures and flow rates.

The first step is to make an aqueous solution containing a glass forming agent and a gas forming agent (hereinafter referred to as a "blowing agent"). In this example the glass forming agent is MSG and the blowing agent is ammonium bicarbonate. The MSG is at a concentration of 200mg/mi and the ammonium bicarbonate at a concentration of 17.4mg/mi. A biological material is then added to the solution. This does not significantly effect the concentrations referred to above.

A Buchi B290 mini spray dryer is set so that gas enters its drying chamber at an inlet gas temperature of 150 C and leaves at approximately 95 C. The drying gas flow rate is set at a nominal value of 600 litres/hour into the drying chamber.

The aqueous solution is introduced into the drying chamber as a fine spray through a 0.7mm nozzle. During a period of between 1 and 1.5 seconds this dries to form a powder which is then collected. The powder is separated from the drying gas in a cyclone atlached to the exhaust of the drying chamber and falls by gravity into a bottle attached to the bottom of the cyclone. During this period the temperature of the particles rises from room temperature (about 21 C) to a theoretical maximum of 95 to 120 C (the outlet temperature). In actual practice the temperature of the dry glass particles does not dwell for any prolonged period at the outlet temperature since they are separated from the hot exhaust gases in the cyclone within a few seconds and collect together in the cooler collection bottle.

As the particles pass though the drying chamber, their temperature initially rises relatively slowly because heat absorbed by the particles is used in the evaporation of the solvent (water). The particles then form into a glass by solvent evaporation, passing through an intermediary stage where they are neither liquid nor glass. In the conditions of the process as described above the particles are at about 60 when they enter this intermediary stage. Continued heating drives off much of the remaining water so that the composition continues to harden through solvent evaporation and soon forms a glass.

Ammonium bicarbonate decomposes when exposed to heat, beginning at 36 C and fully decomposing at 60 C. The products of decomposition are 21.5% ammonia, 5.7% carbon dioxide and 22.8% water vapour (data from the Merck Index). Thus, between approximately 60 C and 70 C, just as the particles are in their intermediary state between liquid and glass, the ammonium bicarbonate is decomposing rapidly into ammonia, carbon dioxide and water vapour. At this time, the MSG is soft and pliable. It is semi-solid like thick syrup. Gas bubbles are therefore trapped within the MSG droplets as they enter a glassy state.

Typical moisture content of the spray-dried final product made by the process as described above is around 4% by weight, measured by the Karl Fischer coulometry method. The particles have a mean density of 0.94 (compared to MSG alone which has a density of 1.46). Powder densities can be measured by pycnometry using helium displacement.

Finally, the dried powder, containing the biological material stabilized by the effect of the glassy MSG, is suspended in a non-aqueous mixture of medium chain triglycerides comprised of approximately 60% caprylic (C8, octanoyl) acid and 40% capric (C 10, decanoyl) acid. This mixture is sold under the trade name of "Crodamol GTCC") and is an anhydrous, non-toxic biocompatible liquid having a density approximately equal to the spray dried spheres. The density of the Crodainol GTCC is sufficiently close to that of each of the spheres, to have the effect that once suspended in the liquid, the spheres are all retained in suspension at normal temperatures. The suspension is so physically and biologically stable that it can be used in pre-fihled injectors and stored and transported without refrigeration. Because of the chemical stabilising effect of the MSG, the active ingredient does not deteriorate and, because of the effect on density of the gas voids in the particles, the latter remain indefinitely in suspension. It will be appreciated that, when the suspension has been injected into an human or animal body, the glass particles, being soluble in water, will dissolve in body fluid, thereby releasing the active ingredient.

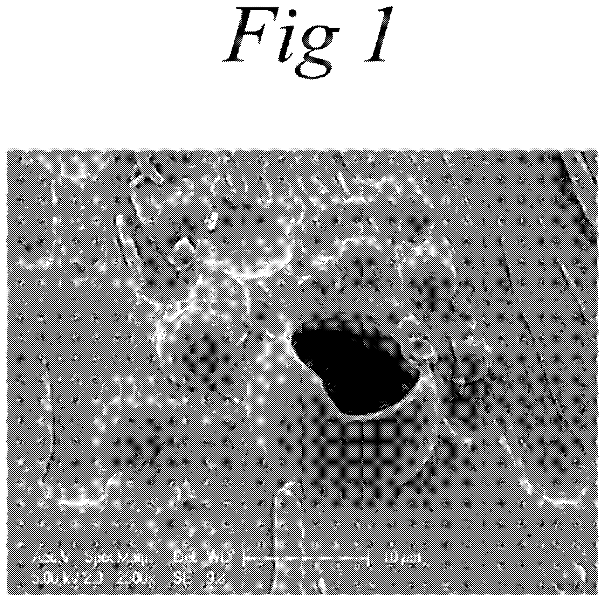

Fig 1 shows a picture, produced using a scanning electron microscope, of spray dried particles manufactured according to the method described above. To inspect the interior structure of the spheres, they were mixed with a carrier liquid which was then frozen and broken in accordance with a standard "freeze-fracture" practice used in microscopy. The broken surface of the frozen carrier forms the background of the picture. It can be seen that the particles are approximately spherical and vary in diameter from about 2 pm to about 15 pm sufficiently small that the suspended particles will pass through the needle of a hypodermic syringe. The size of the particles noted above should be compared with an average particle size of about 3-5 µm that are produced using a similar method but without the ammonium bicarbonate.

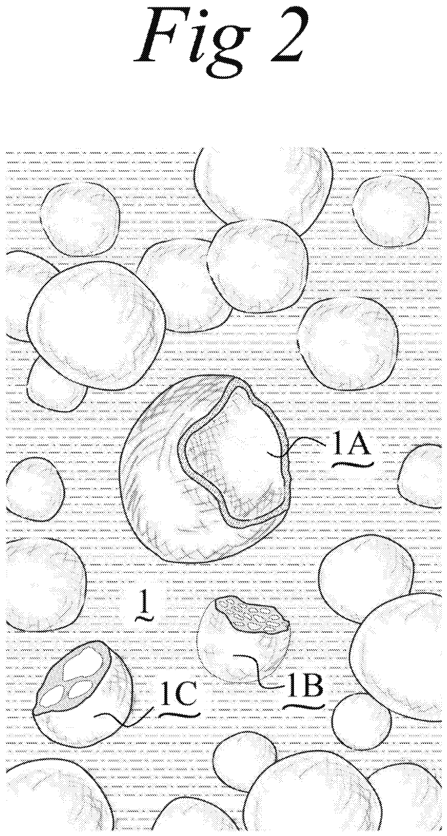

In Fig 1, one of the largest spheres is broken, revealing that it is entirely hollow and has a wall thickness of about 1 pm. It is possible that the smaller spheres may have a similar structure or a more complex structure as illustrated in Fig 2.

Referring to Fig 2, the Crodamol GTCC liquid is indicated at 1 and has, suspended in it, glass particles 2 having a mean density equal to that of the liquid. Particle 2A, shown broken away to reveal its interior, is structured like that shown in Fig 1. Particle 2B, also shown broken away, contains a large number of gaseous voids, forming a structure like a sponge or honeycomb; whilst particle 2C has a structure in- between the extremes of 2A and 2B, having just a few voids. The densities of these different structures will be slightly different but they are all within a range that allows thermodynamic processes at normal temperatures to keep the particles in permanent suspension.

In an alternative process according to the invention, instead of employing pure MSG, a mixture of an equal (O.5M) molar solution of MSG and MSA is formed by the addition of O.935g of MSG and O.865g MSA (total 1.8g) to lOmL of distilled water. Suitable materials include Monosodium L-glutamate monohydrate and monosodium L-aspartate monohydrate from Ajinomoto.

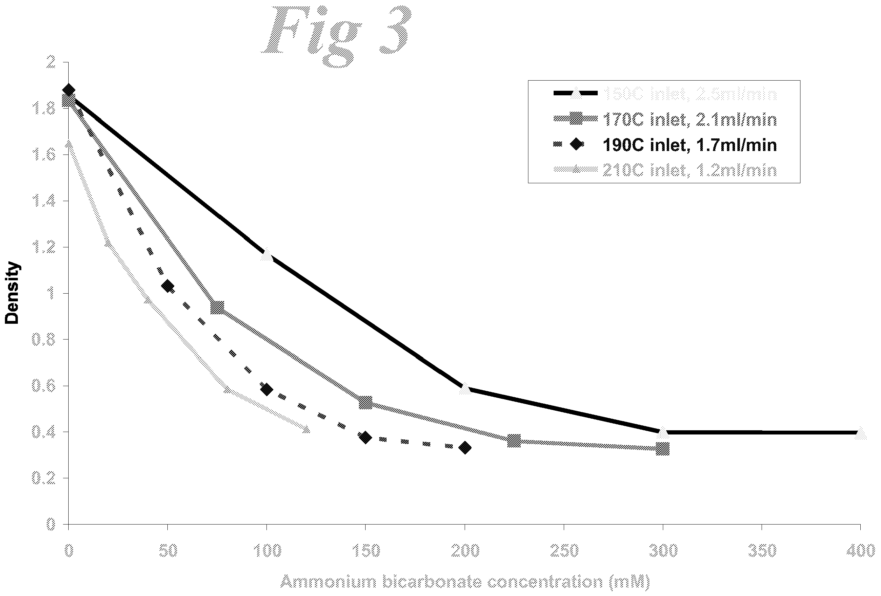

The resulting solution is added to a vaccine material which comprises 2mg of a hepatitis B antigen with 160 mg of aluminium hydroxide adjuvant so as to give a ratio of total stabiliser (i.e. in this case MSA and MSG) to adjuvant of 10.7:1 (1.8g:0.16g). An amount of ammonium bicarbonate within the range shown in Fig 3 is added.

A Buchi B290 mini spray dryer is then used as previously described to form a low density powder.

As, in either of the described processes, the glass is formed from water soluble glass formers, the resulting powder can be rehydrated when needed with the required amount of sterile water.

It will be appreciated that the processes described above are just examples of how the invention may be used. The density of the particles can be precisely controlled, by the addition of a selected quantity of ainmonium bicarbonate, so as to match their density with that of any alternative liquid in which they are to be suspended. Experiments have shown that it is possible to incorporate up to 1.OM (79 mg/mi) ammonium bicarbonate, that latter concentration producing dried spheres with a mean diameter of approximately 20µm and a density of 0.64. Other studies, the results of which are shown on Fig 3, have shown that the effect of varying the ammonium bicarbonate concentration, varying the flow rate of the liquid entering the spray drier and varying the inlet gas temperate of the spray drier is to closely control the density of the microspheres produced.

It is also observed that although, in the particular example described, the gas is released whilst glass is being formed by solvent evaporation, a similar effect may be achievable by using glasses whose transition temperature is matched to the temperature of the gas forming substance (the blowing agent). In such a variation, glass, mixed with the blowing agent would be heated so that it softened, at which point the gas would be released to form the required voids. Subsequent cooling would complete the process.

The techniques described above provide a promising way of adjusting the density of particles so as to match the density of a liquid in which they are subsequently suspended. However, it has been observed that particles produced in accordance with the invention can be of such a low density that they are easily suspended in a gaseous medium and that the invention is therefore of potential application in medications intended for inhalation. The particles, because of their large surface area (including areas exposed to the internal voids), have also been noted as having the ability to dissolve rapidly in aqueous liquids and may therefore be of value in situations where it is desired to store stabilised biological materials in solid stable form and to dissolve them shortly before use.

## Claims

1. A method of making a glassy product for stabilising a biological material comprising the steps of:
i) mixing (a) a first liquid material comprising a biological material and capable of forming a glass, in which the first liquid material comprises a glutamic acid or salt thereof, with (b) a second material which is a blowing agent that undergoes decomposition and forms a gas when heated, in which the second material comprises ammonium bicarbonate; and
ii) causing the first liquid material to form a glass at the same time as the second material forms the gas;
wherein a glassy structure in the form of particles containing the gas is formed in a spray drier.

2. The method according to claim 1, wherein the glass includes monosodium glutamate.

3. The method according to claim 1, wherein the glass includes calcium phosphate.

4. The method according to any one of claims 1 to 3, further comprising the step of forming the glassy structure into particles containing the gas.

5. The method according to any one of claims 1 to 4, further comprising the step of forming the glassy structure into a structural body.

6. The method according to claim 4, comprising the further step of suspending the particles in a non-toxic liquid.

## Patentansprüche

1. Verfahren zur Herstellung eines glasartigen Produkts zur Stabilisierung eines biologischen Materials, das die Schritte aufweist:
i) Mischen (a) eines ersten flüssigen Materials, das ein biologisches Material aufweist und fähig ist, ein Glas zu bilden, wobei das erste flüssige Material eine Glutaminsäure oder ein Salz davon aufweist, mit (b) einem zweiten Material, das ein Treibmittel ist, das zersetzt wird und ein Gas bildet, wenn es erhitzt wird, wobei das zweite Material Ammoniumbikarbonat aufweist; und
ii) Veranlassen des ersten flüssigen Materials, ein Glas zu der selben Zeit zu bilden, wenn das zweite Material das Gas bildet;
wobei eine glasartige Struktur in der Form von Teilchen, die das Gas enthalten, in einem Sprühtrockner gebildet wird.

2. Verfahren nach Anspruch 1, wobei das Glas Mononatriumglutamat aufweist.

3. Verfahren nach Anspruch 1, wobei das Glas Kalziumphosphat aufweist.

4. Verfahren nach einem der Ansprüche 1 bis 3, weiter aufweisend den Schritt des Ausbildens der glasartigen Struktur zu Teilchen, die das Gas enthalten.

5. Verfahren nach einem der Ansprüche 1 bis 4, weiter aufweisend den Schritt des Ausbildens der glasartigen Struktur zu einem Strukturkörper.

6. Verfahren nach Anspruch 4, den weiteren Schritt des Suspendierens der Teilchen in einer ungiftigen Flüssigkeit aufweisend.

## Revendications

1. Procédé de préparation d'un produit vitreux pour stabiliser un matériau biologique comprenant les étapes consistant à :
i) mélanger (a) un premier matériau liquide comprenant un matériau biologique et capable de former un verre, dans lequel le premier matériau liquide comprend un acide glutamique ou un sel de celui-ci, avec (b) un second matériau qui est un agent d'expansion qui subit une décomposition et qui forme un gaz lorsqu'il est chauffé, dans lequel le second matériau comprend du bicarbonate d'ammonium ; et
ii) amener le premier matériau liquide à former un verre en même temps que le second matériau forme le gaz ; dans lequel une structure vitreuse sous la forme de particules contenant le gaz est formée dans un séchoir-atomiseur.

2. Procédé selon la revendication 1, dans lequel le verre comprend du glutamate de monosodium.

3. Procédé selon la revendication 1, dans lequel le verre comprend du phosphate de calcium.

4. Procédé selon l'une quelconque des revendications 1 à 3, comprenant en outre l'étape consistant à former la structure vitreuse en particules contenant le gaz.

5. Procédé selon l'une quelconque des revendications 1 à 4, comprenant en outre l'étape consistant à former la structure vitreuse en un corps structural.

6. Procédé selon la revendication 4, comprenant l'étape supplémentaire consistant à mettre en suspension les particules dans un liquide non toxique.
